# EUROPÄISCHE PATENTSCHRIFT

(11) **EP 0 955 038 B1**
(45) Veröffentlichungstag und Bekanntmachung des Hinweises auf die Patenterteilung: **09.07.2003**
(21) Anmeldenummer: 99107945.0
(22) Anmeldetag: 22.04.1999
(51) Int. Cl.: A61K 7/48

(54) **Kosmetische und dermatologische Zubereitungen mit einem Gehalt an Ceramiden**
Cosmetic and dermatologic compositions containing ceramids
Compositions cosmétiques et dermatologiques contenant des céramides

(30) Priorität: 07.05.1998 DE 19820376
(43) Veröffentlichungstag der Anmeldung: 10.11.1999
(73) Patentinhaber: Beiersdorf Aktiengesellschaft, 20245 Hamburg (DE)
(72) Erfinder: Lanzendörfer, Ghita Dr., 22087 Hamburg (DE); Bleckmann, Andreas, 22926 Ahrensburg (DE); Iwaldow, Karolina, 22309 Hamburg (DE); Rapp, Claudius Dr., 20257 Hamburg (DE); Raschke, Thomas Dr., 25421 Pinneberg (DE)

(56) Entgegenhaltungen:
- EP-A- 0 716 849
- FR-A- 2 675 045

## Beschreibung

Die vorliegende Erfindung betrifft kosmetische und dermatologische Zubereitungen mit einem Gehalt an Ceramid III.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum comeum), den für die Barrierefunktion bedeutenden Teil darstellt. Das heute in der Fachwelt anerkannte Hautmodell von Elias *(P. M. Elias, Structure and Function of the Stratum Corneum Permeability Barrier, Drug Dev. Res.* ***13,*** *1988, 97-105*) beschreibt die Hornschicht als Zwei-Komponenten-System, ähnlich einer Ziegelsteinmauer (Ziegelstein-Mörtel-Modell). In diesem Modell entsprechen die Homzellen (Komeozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die Lipide der Hornschicht bestehen im wesentlichen aus Ceramiden, freien Fettsäuren, Cholesterin und Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Ceramide sind lipophile Amide der allgemeinen Strukturformel 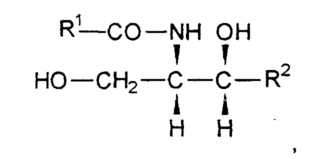 worin R¹ einen langkettigen Alkylrest, der gegebenenfalls einen weiteren ω-Hydroxyacylrest tragen kann, und R² eine der beiden folgenden Strukturen darstellen (wobei x eine Zahl zwischen 8 und 30, in der Regel aber 12 ist):

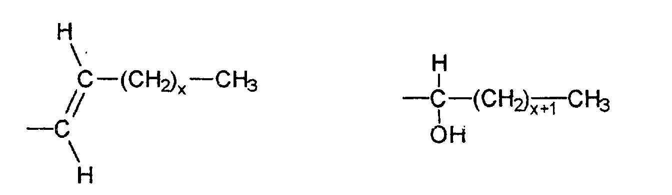

Der ungesättigte Aminodiol-Rest heißt 4-Sphingenin (auch: Sphingosin), weshalb die Ceramide zur Gruppe der Sphingolipide gerechnet werden. In den humanen epidermalen Oberflächenfetten konnten bisher sechs verschiedene Ceramidfraktionen nachgewiesen werden. Eines dieser hautidentischen Ceramide ist [2S,3S,4R]-N-Stearoyl-phytosphingosin, welches auch als Ceramid III oder (nach INCI) Ceramide 3 bezeichnet wird.

Ceramide haben innerhalb der interzellularen Lipidmembran eine wichtige strukturelle Funktion. Sie werden im allgemeinen als essentiell für eine intakte epidermale Barrierefunktion angesehen, und es wird angenommen, daß einer der Gründe für das Erscheinungsbild der trockenen Haut ein Mangel an Ceramiden in den interzellularen Lipiden ist. Es hat daher nicht an Versuchen gefehlt, topische Zubereitungen mit einem Gehalt an Ceramiden zur Pflege insbesondere der trockenen Haut herzustellen.

Zwar kennt der Stand der Technik eine Reihe von Zubereitungen mit einem Gehalt an Ceramiden. Allerdings sind Ceramide nur schwer in größeren Mengen in kosmetische oder dermatologische Formulierungen einzuarbeiten, da sie aus diesen leicht auskristallisieren. Dies ist insbesondere deswegen nachteilig, weil die Wirksamkeit der Ceramide durch das Auskristallisieren herabgesetzt wird. Insbesondere Ceramid III ist in den gängigen kosmetischen Ölen schlecht löslich und auch seine Mischbarkeit mit festen kosmetischen Inhaltsstoffen ist begrenzt.

Aufgabe der vorliegenden Erfindung war, die Nachteile des Standes der Technik zu beseitigen und stabile hautpflegende kosmetische und/oder dermatologische Mittel zur Verfügung zu stellen, welche einen wirksamen Gehalt an Ceramid III haben.

Es war überraschend und für den Fachmann nicht vorauszusehen, daß
kosmetische und/oder dermatologische Zubereitungen mit einem wirksamen Gehalt an Ceramid III, dadurch gekennzeichnet, daß sie mindestens zwei Fettalkohole A und B enthalten, welche unabhängig voneinander gewählt werden aus der Gruppe der verzweigten und/oder unverzweigten, gesättigten und/oder ungesättigten Fettalkohole mit einer Kettenlänge von 12 bis 60 Kohlenstoffatomen, wobei sich die Kettenlänge des Fettalkohols A um mindestens 4 Kohlenstoffatome von der Kettenlänge des Fettalkohols B unterscheidet,
den geschilderten Nachteilen des Standes der Technik Abhilfe schaffen würden.

Die erfindungsgemäßen Zubereitungen sind in jeglicher Hinsicht überaus befriedigende Präparate. Es ist erfindungsgemäß möglich, die Einsatzmengen von Ceramiden in kosmetischen oder dermatologischen Zubereitungen gegenüber dem Stande der Technik deutlich zu erhöhen und wirksame und stabile Zubereitungen herzustellen, welche größere Mengen an Ceramid III enthalten.

Die französische Offenlegungsschrift FR 2 675 045 beschreibt kosmetische Zubereitungen, die neben Shingolipiden, darunter auch Ceramiden, Fettalkohole enthalten, nämlich Cetylalkohol und 3,7,11,15-Tetramethyl-1,2,3-trihydroxyhexadecane. N-Stearoyl-Phytosphingosin wird in diesem Dokument nicht offenbart.

Zwar offenbart die Europäische Patentanmeldung EP 716 849 in den Beispielen 1 und 2 Rezepturen, welche zwei Fettalkohole - namentlich Cetyl- und Stearylalkohol - enthalten. Allerdings sind diese beiden Fettalkohole gängige Bestandteile kosmetischer Zubereitungen und nicht dazu geeignet, Ceramid III auch in hohen Mengen zu lösen. Der Stand der Technik konnte daher nicht den Weg zur vorliegenden Erfindung bereiten.

Kosmetische und dermatologische Zubereitungen im Sinne der vorliegenden Erfindung enthalten vorzugsweise insgesamt 0,01 bis 20 Gew.-%, insbesondere 0,1 bis 10 Gew.-%, besonders bevorzugt 0,5 bis 6 Gew.-% der beiden Fettalkohole A und B, jeweils bezogen auf das Gesamtgewicht der Zubereitungen. Vorteilhaft wird das Gewichtsverhältnis von Fettalkohol A zu Fettalkohol B in der Fettalkoholmischung aus dem Bereich von 0,01 : 1 bis 1 : 0,01 gewählt.

Es ist vorteilhaft im Sinne der vorliegenden Erfindung, Fettalkohol A und Fettalkohol B unabhängig voneinander aus der Gruppe Myristylalkohol (CH₃(CH₂)₁₂CH₂OH), Cetylalkohol (CH₃(CH₂)₁₄CH₂OH), Stearylalkohol (CH₃(CH₂)₁₆CH₂OH) und Behenylalkohol (CH₃(CH₂)₂₀CH₂OH) zu wählen. Besonders bevorzugt sind Zubereitungen, in denen einer der beiden Fettalkohole A oder B Behenylalkohol ist.

Kosmetische und dermatologische Zubereitungen im Sinne der vorliegenden Erfindung enthalten vorzugsweise 0,01 bis 2 Gew.-% Ceramid III. Vorteilhaft wird das Gewichtsverhältnis von Fettalkoholmischung zu Ceramid III aus dem Bereich von 1:2 bis 50:1 gewählt.

Die erfindungsgemäßen Zubereitungen können wie üblich zusammengesetzt sein und zur Behandlung, der Pflege und der Reinigung der Haut und/oder der Haare und als Schminkprodukt in der dekorativen Kosmetik dienen. Entsprechend können sie, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden.

Zubereitungen zur Behandlung und Pflege der Haut, insbesondere der trockenen Haut werden bevorzugt.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Enthalten die erfindungsgemäßen kosmetischen und dermatologischen Zubereitungen UV-B-Filtersubstanzen, können diese öllöslich oder wasserlöslich sein. Erfindungsgemäß vorteilhafte öllösliche UV-B-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UV-B-Filter, die in den erfindungsgemäßen Zubereitungen verwendet werden können, soll selbstverständlich nicht limitierend sein.

Es kann auch von Vorteil sein, in erfindungsgemäßen kosmetischen und dermatologischen Zubereitungen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die UV-A-Filter enthalten, sind Gegenstand der Erfindung.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W) oder Öl-in-Wasser-in-Öl (O/WO), ein Gel, eine Hydrodispersion, einen festen Stift, eine Salbe oder auch ein Aerosol darstellen.

Es ist auch möglich und vorteilhaft im Sinne der vorliegenden Erfindung, die erfindungsgemäßen Wirkstoffe in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die jeweils einzusetzenden Mengen an kosmetischen oder dermatologischen Trägerstoffen und Parfüm können in Abhängigkeit von der Art des jeweiligen Produktes vom Fachmann durch einfaches Ausprobieren leicht ermittelt werden.

Insbesondere können die erfindungsgemäßen kosmetischen und dermatologischen Zubereitungen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Vorteilhaft können erfindungsgemäße Zubereitungen außerdem als Grundlage für kosmetische Desodorantien bzw. Antitranspirantien eingesetzt werden.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Hingegen kann durch die Verwendung antimikrobieller Stoffe in kosmetischen Desodorantien die Bakterienflora auf der Haut reduziert werden. Dabei sollten im Idealfall nur die Geruch verursachenden Mikroorganismen wirksam reduziert werden. Der Schweißfluß selbst wird dadurch nicht beeinflußt, im Idealfall wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

### Beispiele:

| **Beispiel 1** | |
|---|---|
| Ceramid III | 0,3 % |
| Cetylalkohol | 2,0 % |
| Behenylalkohol | 2,0 % |
| Carbomer | 0,5 % |
| NaOH (45%ige Lösung in Wasser) | 0,25 % |
| Glycerin | 3,0 % |
| Konservierungsmittel | q.s. |
| Wasser | ad 100 |

| **Beispiel 2** | |
|---|---|
| Mineralöl | 3,0 % |
| Ceramid III | 0,5 % |
| Stearylalkohol | 4,0 % |
| Behenylalkohol | 2,0 % |
| Glycerin | 3,0 % |
| Carbomer | 0,5 % |
| NaOH (45%ige Lösung in Wasser) | 0,25 % |
| Konservierungsmittel | q.s. |
| Wasser | ad 100 |

| **Beispiel 3** | |
|---|---|
| Saccharosestearat | 3,0 % |
| Stearylalkohol | 3,0 % |
| Myristylalkohol | 1,0 % |
| Ceramid III | 0,5 % |
| Glycerin | 3,0 % |
| Miglyol 812¹ ® | 6,0 % |
| Mineralöl | 2,0 % |
| Isopropylpalmitate | 3,0 % |
| Panthenol | 0,5 % |
| Konservierungsmittel | q.s. |
| Wasser | ad 100 |

| | |
|---|---|
| ¹ Capryl-Caprinsäure-Triglycerid | |

| **Beispiel 4** | |
|---|---|
| Polyglyceryl-3-Methylglucosedistearat | 2,0 % |
| Ceramid III | 0,2 % |
| Squalan | 3,0 % |
| Jojobaöl | 3,0 % |
| Octyldodecanol | 1,0 % |
| Stearylalkohol | 0,5 % |
| Behenylalkohol | 1,0 % |
| Glycerin | 5,0 % |
| Carbomer | 0,6 % |
| NaOH (45%ige Lösung in Wasser) | 0,3 % |
| Konservierungsmittel | q.s. |
| Wasser | ad 100 |

| **Beispiel 5** | |
|---|---|
| Glycerylmonostearat | 3,0 % |
| PEG-40 Stearat | 0,8 % |
| Glycerin | 3,0 % |
| Mineralöl | 10,0% |
| Isopropylpalmitat | 4,0 % |
| Cyclomethicon | 4,0 % |
| Myristylalkohol | 1,0 % |
| Behenylalkohol | 0,5 % |
| Ceramid III | 0,6 % |
| Carbomer | 0,2 % |
| NaOH (45%ige Lösung in Wasser) | 0,1 % |
| Konservierungsmittel | q.s. |
| Wasser | ad 100 |

| **Beispiel 6** | |
|---|---|
| Glycerylisostearat | 1,0 % |
| Lanolinalkohol | 2,0 % |
| Dimethicone | 2,0 % |
| Sonnenblumenöl | 2,0 % |
| Vaseline | 5,0 % |
| Isohexadecan | 3,0 % |
| Ceresin | 2,5 % |
| Cetylalkohol | 0,3 % |
| Behenylalkohol | 0,3 % |
| Ceramid III | 0,3 % |
| Butylenglycol | 3,0 % |
| Carbomer | 0,2 % |
| NaOH (45%ige Lösung in Wasser) | 0,1 % |
| Konservierungsmittel | q.s. |
| Wasser | ad 100 |

| **Beispiel 7** | |
|---|---|
| Cetyldimethicone Copolyol | 2,0 % |
| Glycerin | 3,0 % |
| Dimethicone | 50,0 % |
| Cyclomethicone | 5,0 % |
| Cetylalkohol | 2,0 % |
| Behenylalkohol | 1,0 % |
| Ceramid III | 0,1 % |
| NaCI | 0,5 % |
| Konservierungsmittel | q.s. |
| Wasser | ad 100 |

| **Beispiel 8** | |
|---|---|
| Stearylalkohol | 0,6 % |
| Behenylalkohol | 1,0 % |
| Ceramid III | 0,2 % |
| Dimethicone Copolyol | 2,0 % |
| PEG-8 Bienenwachs | 8,0 % |
| Isopropylpalmitat | 5,0 % |
| Shea Butter | 2,0 % |
| Hydrierte Cocosfettglyceride | 2,0 % |
| Carbomer | 0,4 % |
| NaOH 45%ig | 0,2 % |
| Konservierungsmittel | q.s. |
| Wasser | ad 100 |

## Patentansprüche

1. Kosmetische und/oder dermatologische Zubereitungen mit einem wirksamen Gehalt an [2S,3S,4R]-N-Stearoyl-phytosphingosin (Ceramid III), **dadurch gekennzeichnet, daß** sie mindestens zwei Fettalkohole A und B enthalten, welche unabhängig voneinander gewählt werden aus der Gruppe der verzweigten und/oder unverzweigten, gesättigten und/oder ungesättigten Fettalkohole mit einer Kettenlänge von 12 bis 60 Kohlenstoffatomen, wobei sich die Kettenlänge des Fettalkohols A um mindestens 4 Kohlenstoffatome von der Kettenlänge des Fettalkohols B unterscheidet.

2. Kosmetische und/oder dermatologische Zubereitungen nach Anspruch 1, **dadurch gekennzeichnet, daß** zusätzlich kosmetische oder dermatologische Hilfs-, Zusatz- und/oder Wirkstoffe enthalten sind.

3. Kosmetische und/oder dermatologische Zubereitungen nach einem der Ansprüche 1 oder 2, **dadurch gekennzeichnet, daß** Fettalkohol A und Fettalkohol B unabhängig voneinander aus der Gruppe Myristylalkohol, Cetylalkohol, Stearylalkohol und Behenylalkohol gewählt werden.

4. Kosmetische und/oder dermatologische Zubereitungen nach einem der vorhergehenden Ansprüche, **dadurch gekennzeichnet, daß** der Gehalt der beiden Fettalkohole A und B zusammen insgesamt 0,01 bis 20 Gew.-%, insbesondere 0,1 bis 10 Gew.-%, besonders bevorzugt 0,5 bis 6 Gew.-%, jeweils bezogen auf das Gesamtgewicht der Zubereitungen beträgt.

5. Kosmetische und/oder dermatologische Zubereitungen nach einem der vorhergehenden Ansprüche, **dadurch gekennzeichnet, daß** das Gewichtsverhältnis von Fettalkohol A zu Fettalkohol B aus dem Bereich von 0,01 : 1 bis 1 : 0,01 gewählt wird.

6. Kosmetische und/oder dermatologische Zubereitungen nach einem der vorhergehenden Ansprüche, **dadurch gekennzeichnet, daß** der Gehalt an Ceramid III 0,01 bis 2 Gew.-%, bezogen auf das Gesamtgewicht der Zubereitungen, beträgt.

7. Kosmetische und/oder dermatologische Zubereitungen nach einem der vorhergehenden Ansprüche, **dadurch gekennzeichnet, daß** das Gewichtsverhältnis von Fettalkoholmischung zu Ceramid III aus dem Bereich von 1 : 2 bis 50 : 1 gewählt wird.

## Claims

1. Cosmetic and/or dermatological preparations with an effective content of [2S,3S,4R]-N-stearoyl-phytosphingosine (ceramide III), **characterized in that** they comprises at least two fatty alcohols A and B which, independently of one another, are chosen from the group of branched and/or unbranched, saturated and/or unsaturated fatty alcohols with a chain length of from 12 to 60 carbon atoms, where the chain length of the fatty alcohol A differs from the chain length of the fatty alcohol B by at least 4 carbon atoms.

2. Cosmetic and/or dermatological preparations according to Claim 1, **characterized in that** cosmetic or dermatological auxiliaries, additives and/or active ingredients are additionally present.

3. Cosmetic and/or dermatological preparations according to either Claim 1 or 2, **characterized in that** fatty alcohol A and- fatty alcohol B are chosen, independently of one another, from the group consisting of myristyl alcohol, cetyl alcohol, stearyl alcohol and behenyl alcohol.

4. Cosmetic and/or dermatological preparations according to one of the preceding claims, **characterized in that** the content of the two fatty alcohols A and B together is in total 0.01 to 20% by weight, in particular 0.1 to 10% by weight, particularly preferably 0.5 to 6% by weight, in each case based on the total weight of the preparations.

5. Cosmetic and/or dermatological preparations according to one of the preceding claims, **characterized in that** the weight ratio of fatty alcohol A to fatty alcohol B is chosen from the range from 0.01:1 to 1:0.01.

6. Cosmetic and/or dermatological preparations according to one of the preceding claims, **characterized in that** the content of ceramide III is 0.01 to 2% by weight, based on the total weight of the preparations.

7. Cosmetic and/or dermatological preparations according to one of the preceding claims, **characterized in that** the weight ratio of fatty alcohol mixture to ceramide III is chosen from the range from 1:2 to 50:1.

## Revendications

1. Préparations cosmétiques et/ou dermatologiques ayant une teneur efficace en [2S,3S,4R]-N-stéaroyl-phytosphingosine (céramide III), **caractérisées en ce qu'**elles contiennent au moins deux alcools gras A et B qui sont choisis, indépendamment l'un de l'autre, dans le groupe des alcools saturés et/ou insaturés, ramifiés et/ou non ramifiés, ayant une longueur de chaîne de 12 à 60 atomes de carbone, la longueur de chaîne de l'alcool gras A différant d'au moins 4 atomes de carbone de la longueur de chaîne de l'alcool gras B.

2. Préparations cosmétiques et/ou dermatologiques selon la revendication 1, **caractérisées en ce qu'**elles contiennent en outre des adjuvants, additifs et/ou substances actives cosmétiques ou dermatologiques.

3. Préparations cosmétiques et/ou dermatologiques selon la revendication 1 ou 2, **caractérisées en ce que** l'alcool gras A et l'alcool gras B sont choisis, indépendamment l'un de l'autre, dans le groupe constitué par l'alcool myristylique, l'alcool cétylique, l'alcool stéarylique et l'alcool béhénylique.

4. Préparations cosmétiques et/ou dermatologiques selon l'une quelconque des revendications précédentes, **caractérisées en ce que** la teneur en les deux alcools gras A et B ensemble va au total de 0,01 à 20 % en poids, en particulier de 0,1 à 10 % en poids, de façon particulièrement préférée de 0,5 à 6 % en poids, chaque fois par rapport au poids total des préparations.

5. Préparations cosmétiques et/ou dermatologiques selon l'une quelconque des revendications précédentes, **caractérisées en ce que** le rapport pondéral de l'alcool gras A à l'alcool gras B est choisi dans la plage allant de 0,01:1 à 1:0,01.

6. Préparations cosmétiques et/ou dermatologiques selon l'une quelconque des revendications précédentes, **caractérisées en ce que** la teneur en céramide III va de 0,01 à 2 % en poids, par rapport au poids total des préparations.

7. Préparations cosmétiques et/ou dermatologiques selon l'une quelconque des revendications précédentes, **caractérisées en ce que** le rapport pondéral du mélange d'alcools gras au céramide III est choisi dans la plage allant de 1:2 à 50:1.
